# EUROPEAN PATENT APPLICATION

(11) **EP 2 258 844 A1**
(43) Date of publication of application: **08.12.2010**
(21) Application number: 09161442.0
(22) Date of filing: 28.05.2009
(51) Int. Cl.: C12N 15/09, C12N 15/85, A61K 48/00

(54) **Tumour-specific bacterial promoter elements**

(71) Applicant: HELMHOLTZ ZENTRUM FÜR INFEKTIONSFORSCHUNG, 38124 Braunschweig (DE)
(72) Inventor: Weiß, Siegfried Dr., 38124 Braunschweig (DE); Bartels, Sara, 38124 Braunschweig (DE); Lößner, Holger Dr., 63225 Langen (DE); Deyneko, Igor, 38124 Braunschweig (DE); Bumann, Dirk, 4125 Riehen (CH); Westphal-Daniel, Kathrin dr., 38124 Braunschweig (DE)
(74) Representative: Taruttis, Stefan Georg

(57) **Abstract**

The invention relates to bacterial promoter elements which constitute or which are comprised in promoter regions and which confer tumour specificity to the promoter region activity, resulting in transcription of a transgene, which can be a heterologous or a homologous gene, which transgene is functionally arranged downstream of the promoter region at presence of a host bacterium in tumour tissue, while essentially conferring inactivity of the promoter region and no transcription at presence of the host bacterium in non-tumour tissue. Accordingly, the invention relates to bacterial promoter regions containing these tumour specific promoter elements.

SEQUENCE LISTING

<110>  Helmholtz-Zentrum fuer Infektionsforschung GmbH

<120>  Tumor-specific bacterial promotor elements

<130>  G1023EP

<160>  28

<170>  PatentIn version 3.5

<210>  1
<211>  21
<212>  DNA
<213>  Artificial Sequence

<220>
<223>  SEQ ID NO. 1, wherein R = G or A, Y = T or C, M = A or C, K = G
       or T, W = A or T, S = G or C,

       B = T or G or C, V = A or G or C, H = A or T or C, D = A or T or
       G,

N = A or T or G or C


<220>
<221>  misc_feature
<222>  (3)..(3)
<223>  n is a, c, g, or t

<220>
<221>  misc_feature
<222>  (15)..(15)
<223>  n is a, c, g, or t

<400>  1
yhnydtkttw ttwanasrwa m                                                21


<210>  2
<211>  21
<212>  DNA
<213>  Artificial Sequence

<220>
<223>  SEQ ID NO. 2

<400>  2
cagtatttta tttaaaggta a                                                21


<210>  3
<211>  21
<212>  DNA
<213>  Artificial Sequence

<220>
<223>  SEQ ID NO. 3, wherein R = A or G

<400>  3
ccatatttta tttaraggta a                                                21


<210>  4
<211>  21
<212>  DNA
<213>  Artificial Sequence

<220>
<223>  SEQ ID NO. 4

<400>  4
ccatatttta tttagaggta a                                                21


<210>  5
<211>  472
<212>  DNA
<213>  Artificial Sequence

<220>
<223>  SEQ ID NO. 5 (4ydiH)

<400>  5
gatctttgat gaacagggac gtctgtgctg ttcttcgcgt cttactacag cgattgtgtg      60

acgttgtcac cataaaagtg tgatgcatat tgcttttggt caaaatgtgc gcagaaaaag     120

gtgatatact gtgcacgttt acacataagc gaggatgata tgtctactga tttggaccca     180

acccaactgg cgattgaatt tttacgccgt gataaaaccg aactttctcc cgcgcagtat     240

ttgaagcgtt taaaacagct ggagttagag tttgccgatc tcctcaccct ctcagcaacc     300

gaactgaaag aagagatcta tttcgcctgg cggttgggcg tgcattaata aagtctgtgt     360

aggccggata aggcgttagc cgccatccgg ctatgttacc aggcaatctc accagacctt     420

gtccccaggt aatagataac ccccttgatt ttgtttaaaa aaaatacaga tc              472


<210>  6
<211>  21
<212>  DNA
<213>  Artificial Sequence

<220>
<223>  SEQ ID NO. 6 (tumor-specific promotor element of 4ydiH)

<400>  6
ttttgtttaa aaaaaataca g                                                21


<210>  7
<211>  1152
<212>  DNA
<213>  Artificial Sequence

<220>
<223>  SEQ ID NO. 7 (t48gIpT)

<400>  7
gatcgatggc ttcagcgcca atgcctgccg cttcacaggc gcgaataaag gtggcctgaa      60

acgcaagatc atcttccggc aacgtaataa acagaccatc cgtcggttcg acgcaatggc     120

gggcgatccg ttttaaaatc tggttttcac taatgcactc gcgcgcggat tccgcgtcgg     180

taaccgcata acgagcgccg ctgtgcagca gcccgtggtt acgcccggtc gcgccggtcg     240

ctatatcatg ccgctccacc agaatgacac gtaaaccgcg cagcgcgcag tcgcgggcga     300

tccctgcgcc tgttgctcca ccgccaatga taatcacgtc acttgtttgc gagtcgcgag     360

ttttcattgt ttttcctcac agttcgtttt ttatcattta gccatacaaa tcatatgtaa     420

tgtttgattt cgcgcataat cgctcactat tcgaaaatga aacgtgattt cgtgcgcctt 480

tctgaacatt agtcataaat ctgtaacaat atgtgctgta attcacatta acgtgacgta 540

tctttactta acatcgcggc cacactggga gcagcggggt tgtttgaacg aactgcggtg 600

tttacctcta aataaaatat gggccacgga ggctacaata tgttgagtat ttttaaacca 660

gcgccgcata aagcgcgctt gccagcggcg gagattgatc cgacctatcg ccgattacgc 720

tggcagattt tcctggggat attctttggc tatgccgcgt attatctggt gcgcaagaac 780

tttgccctcg cgatgcccta cctggtagaa cagggttttt cacgcggcga tctgggcttt 840

gcgctgtccg ggatttccat cgcttatggt ttttcgaaat ttataatggg ttcggtgtcc 900

gatcgctcga atccgcgcgt ttttctgccg gcagggttga ttctggccgc agcagtcatg 960

ttgtttatgg gctttgtgcc gtgggcgaca tccagcatcg ccgtgatgtt tgtactgttg 1020

ttcctttgcg gctggttcca ggggatgggg tggccgccgt gcggtcgtac gatggttcac 1080

tggtggtcgc agaaagagcg cggcggcatt gtgtcggtct ggaacggcgc gcataacgtc 1140

ggcggcggga tc 1152

<210> 8
<211> 21
<212> DNA
<213> Artificial Sequence

<220>
<223> SEQ ID NO. 8 (tumor-specific promotor element of 48GIpT)

<400> 8
ttacctctaa ataaaatatg g 21

<210> 9
<211> 1093
<212> DNA
<213> Artificial Sequence

<220>
<223> SEQ ID NO. 9 (92yhjU)

<400> 9
gatcgcttta caggcgaacc cccttctcaa tcgtcatcgg atgatttacg gtacgttaac 60

ggcggaactt tccacaaccg tacatgcgct ggcactgcat acttataccc tcaaagagtg 120

ggaaggattg caggatacta tcttcgcgtc gcctccttgt cgcggcgcgg gaagcatcgc 180

gtagcaaaac ggatttgcaa ctacctccgc ttttccagta tgttgctaca gaattatgtg 240

aaaacggcct gcgggccgtt ttgttttgtc tgaattttga gcgtgtcgta cagtattcag 300

acaaaaatta gccgagaatt gtgaaaaccg ccgcagcatc gcacaatcac cgttctcgac 360

tcacaaaagt gatgccgcta taatgcgccg tcttatatat gaacgtcttc gggatgattc 420

tgacgacagg gaatgtgatt gattacgaga acatcccggt tccgcgaagc aaatagcacg 480

tgcttgcgga gtagagttga ccgagcactg tgattttttg aggtaacaag atgcaagttt 540

cagttgaaac cactcagggg ccttggccgc cgtgtaacga ttacaatcgc tgctgacagc 600

atcgagaccg ctgtaaaaag cgagctggtc aacgtagcga aaaaagtacg tattgacggc 660

ttccgtaaag gcaaagtacc gatgaatatc gtcgctcagc gttatggcgc ttctgttcgc 720

caggacgtgc tgggcgatca ttccctggaa cgccccgctt tcacgctgcc tgacgcttat 780

tgaaagcgtg caggggcagc agttcagccg ttacgtaccg gaagacatca ccacgctact 840

gtcgatgacg cagccgttga aactgcgcgg ttttcagccg tgggatacct tctgcgatgc 900

catccatacg atgatgagca acaccctgct ccccgccgac gggaaaggcg ttctggtcgc 960

gctgcgcccg gtgccgggca ttcgggttga gcaggcgtta acattatgtc ggccaaacag 1020

ggccggcgat attatgacca tcggcggcaa ccgtctggaa aggtggttat cattctgccg 1080

gggtctaacg atc 1093

<210> 10
<211> 21
<212> DNA
<213> Artificial Sequence

<220>
<223> SEQ ID NO. 10 (tumor-specific promotor element of 92yhjU)

<400> 10
cactgtgatt ttttgaggta a 21

<210> 11
<211> 715
<212> DNA
<213> Artificial Sequence

<220>
<223> SEQ ID NO. 11 (135dniR)

<400> 11
gatcccggcg aagcggcccc cgtcttaaag gccattgctg aacataagtg gatgccggag 60

gcgatttttt tgacgcatca ccatcacgac catgttggcg gagtcaaaga gctgttgcaa 120

cacttcccgc aaatgacggt ttatggaccg gcggaaacgc aagacaaggg agcaacccat 180

cttgttggcg atggcgatac tattcgcgtt ttaggcgaga aatttactct ttttgccacg 240

ccgggccaca cgttaggaca cgtctgttac tttagccgcc cttacttatt ctgcggcgac 300

acgctgtttt ccggcggctg tggtcgactg tttgaaggca cgccatcaca gatgtatcag 360

tcacttatga aaattaactc tctgcctgac gacacgctca tttgctgcgc tcacgaatac 420

actttagcta acattaagtt cgcattgagc atacttccgc acgattcgtt cataaatgaa 480

tattatcgta aagttaaaga gttacgtgta aaaaaacaaa tgacattacc cgttattctt 540

aaaaatgagc gtaagattaa tcttttttta agaactgaag atattgattt aattaacgaa 600

ataaacaaag aaacaatatt gcaacaacca gaagcgcgtt ttgcatggtt aaggtcaaag 660

aaagacacgt tctgataatt cttacttgtc attcgctaac ttcgccgtta tgatc 715

<210> 12
<211> 21
<212> DNA
<213> Artificial Sequence

<220>
<223>  SEQ ID NO. 12 (tumor-specific promotor element of 134dniR)

<400>  12
ttacgtgtaa aaaaacaaat g                                                21


<210>  13
<211>  466
<212>  DNA
<213>  Artificial Sequence

<220>
<223>  SEQ ID NO. 13 (154mdh)

<400>  13
gatccggatt acgcaaatta aatgcataaa agccaaaatt gcgcgactcc gcattcttga      60

tgagtgagga ttgtaatcat tgaatttgtg aattaaggtc gccgccgcgg agcaatagac     120

acttagctaa tcatataata aggagtttag gatgaaagtc gcagtcctcg gcgctgctgg     180

tggtatcggt caggcgctgg cattactttt aaaaaaccaa ctgccttcag gttcagaact     240

ctccctgtac gacatcgctc cagtgactcc cggtgtggcc gttgatttga gccacatccc     300

caccgctgta aaaatcaaag gtttctccgg tgaagacgca accccggcgc ttgaaggcgc     360

tgacgtagta ctgatttctg cgggtgtggc gcgtaagccg ggtatggacc gttccgacct     420

gtttaacgtt aacgccggca tcgtgaaaaa cctggtgcag cagatc                    466


<210>  14
<211>  21
<212>  DNA
<213>  Artificial Sequence

<220>
<223>  SEQ ID NO. 14 (tumor-specific promotor element of 154mdh)

<400>  14
ttacttttaa aaaaccaact g                                                21


<210>  15
<211>  564
<212>  DNA
<213>  Artificial Sequence

<220>
<223>  SEQ ID NO. 15 (156yeel)

<400>  15
gatcgcaagg tgaaaatgag cccaaccctg gacaggaagc gttgagcttt tcgatgtgcg      60

ccagttaaaa ttctggcgtt tttttctcac cgaattttct cattttttct caacgtgatt     120

ttcatcacta taagaaaatc acgtaagtgc ttgaatagtg gcggagagag agggattcga     180

accctcggcg gagttacccc cgcaacggtt ttcgagaccg gtccgttcag ccgctccggc     240

atctctccgt atattgcaat gatgccaggt aatttggcat tttaacagac cctattcggg     300

taattttgtt caagtgacga gtttacgagc aaaacgatga ttaagtggcc ctggaaagca     360

caagaaataa cccagaacga agactggccg tgggatgatg cgctggctat acctcttctg     420

gtaaacctca ccgcgcaaga acaggctcgg cttattgcgc tagccgaacg ttttttgcag     480

cagaaaagac tggtagcgct acagggattt gagctcgact cgttaaaaag tgcacgtatt 540

gcgttaattt tttgcttacc gatc 564

<210> 16
<211> 21
<212> DNA
<213> Artificial Sequence

<220>
<223> SEQ ID NO. 16 (tumor-specific promotor element of 156yeel)

<400> 16
tctcattttt tctcaacgtg a 21

<210> 17
<211> 441
<212> DNA
<213> Artificial Sequence

<220>
<223> SEQ ID NO. 17 (172frdA)

<400> 17
gatcggcaaa gaaacgacgg atttccgcca taatcgccgc acgttttaat aaattgggga 60

tggacgcgct cggctgccag gttgccgttt cgctcatgat tctttctcca gtttaagaca 120

aggtcacgaa gtctactcgc aacgcgcggg cgaaacaaat tttgcgcagg cgtatcgggc 180

gccttctgga gggtaaaaaa agtgatttca gatggtttag taattaaatt aatcaaaatc 240

aatgataatt catccctctg atacgctaaa aaaatcgaac acgtcaaatt tccctcacat 300

ccctgagact atactgttgt acccataaag gagcagtgga aacgcattca tacttcgcag 360

aacccagagg ctttatctgg ctgcgcgagg gtgaaattac aataatctgg aggaatgtcg 420

tgcaaacctt tcaagccgat c 441

<210> 18
<211> 21
<212> DNA
<213> Artificial Sequence

<220>
<223> SEQ ID NO. 18 (tumor-specific promotor element of 172frdA)

<400> 18
gcacgtttta ataaattggg g 21

<210> 19
<211> 614
<212> DNA
<213> Artificial Sequence

<220>
<223> SEQ ID NO. 19 (185pfkA)

<400> 19
gatcgcgcgc tgcccgatgc ggaacgacag gaaattattg atatcgtgac gtcatggccg 60

ggagtcagcg gcgcgcacga tctccgcacg cggcagtcag ggccgactcg ctttattcag 120

attcatttgg aaatggaaga taatctgccg ctcgttcagg cgcattttgt ggctgaccag 180

attcatttgg aaatggaaga taatctgccg ctcgttcagg cgcattttgt ggctgaccag 240

tgttcagtcg ttcccaggga aggcaggaag ttcgagcttg tataattgat tgttaaaaag 300

tgagccaggc cagcattttg tgtataaatt accgccattt ggcctgacct gaatcaattc 360

agcaggaagg gattgttata ctatctgtat attcgttgga tcgtttcgaa gtgcgaaatc 420

ggcttccggc aatagatttc attttgcatt ccaaagttca gaggtagtca tgattaagaa 480

aatcggtgtg ttgacaagcg gcggtgatgc gccgggcatg aacgcggcaa tccgcggtgt 540

tgtgcgcgca gcgttgacgg aagggctgga agtcatgggc atttatgacg gctatctggg 600

cctgtatgaa gatc 614

<210> 20
<211> 21
<212> DNA
<213> Artificial Sequence

<220>
<223> SEQ ID NO. 20 (tumor-specific promotor element of 185pfkA)

<400> 20
cagcattttg tgtataaatt a 21

<210> 21
<211> 437
<212> DNA
<213> Artificial Sequence

<220>
<223> SEQ ID NO. 21 (212pflE)

<400> 21
gatcggttcg ctacaggcaa tggaggccat taagctactg gcgcattacg gtcagcctgc 60

cagcggaaaa atcgtcatgt acgatgcgat gacctgtcag ttccgcgaaa tgaagttaat 120

gcgcaacccc ggctgtgagg tctgcgggca gtagccactt tacggataaa cataccaaag 180

cacggtttat ggccgtgctg tatcaacaga taattactgt catcagacgc aaagccagct 240

tacccaagct ccgactgacg atcggctcaa gcagcgcggc atcatcttaa acgccatacc 300

acaacctcaa accgtgatgt tgtacctcat ttaacgcctc tttgtcagaa cctctccatt 360

cgttgacgca catcaagata gctttcattc gaaagtaatt taatctttat atgaaataag 420

agaggccgtt tatgatc 437

<210> 22
<211> 20
<212> DNA
<213> Artificial Sequence

<220>
<223> SEQ ID NO. 22 (tumor-specific promotor element of 212pflE)

<400> 22
taatctttat atgaaataag 20

<210> 23
<211> 556
<212> DNA
<213> Artificial Sequence

<220>
<223> SEQ ID NO. 23 (nirB)

<400> 23
gatcgcagca aacgaaagtg gcctcgccta agctggttaa cttcattctg acctgcggca 60

ccatcggaac aatgctgacc ttcgtcgtca ccggcccgat tgtagcgcac agcggcccac 120

aggcggcgtt actcaccgcg aatggtctgt atgcggtggt ctttgtgatg tgctttgcgc 180

tcggctttgt atcccgtcat cgtcagcata gcgcgccggc tacgcattga taatccttgc 240

cggatggaga catcgccctc cggcactcta tcccccctcc tgacggggta ggcctgttgg 300

tctaaaaacc cctcattttg tatgttattt gtacaaacct gaaaagcctg acaattccgc 360

cacttataaa aatccagaca aatcagccat atacccatta agaggtatat aaaggtgaat 420

ttgatttaca tcaataagcg gggttgctga atcgttaagg taggcggtaa tagaaaagaa 480

atcgaggcaa aaatgagcaa agtcagactc gctattatcg gtaatggtat ggtcggccac 540

cgctttattg aggatc 556

<210> 24
<211> 21
<212> DNA
<213> Artificial Sequence

<220>
<223> SEQ ID NO. 24 (tumor-specific promotor element of 271nirB)

<400> 24
ttgtatgtta tttgtacaaa c 21

<210> 25
<211> 708
<212> DNA
<213> Artificial Sequence

<220>
<223> SEQ ID NO. 25 (301glpA)

<400> 25
gatcgccgcg tgaaaaaccc tgttctacca ggtagggcat cgcgagggca aagttcttgc 60

gcaccagata atacgcggca tagccaaaga atatccccag gaaaatctgc cagcgtaatc 120

ggcgataggt cggatcaatc tccgccgctg gcaagcgcgc tttatgcggc gctggtttaa 180

aaatactcaa catattgtag cctccgtggc ccatatttta tttagaggta aacaccgcag 240

ttcgttcaaa caaccccgct gctcccagtg tggccgcgat gttaagtaaa gatacgtcac 300

gttaatgtga attacagcac atattgttac agatttatga ctaatgttca gaaaggcgca 360

cgaaatcacg tttcattttc gaatagtgag cgattatgcg cgaaatcaaa cattacatat 420

gatttgtatg gctaaatgat aaaaaacgaa ctgtgaggaa aaacaatgaa aactcgcgac 480

tcgcaaacaa gtgacgtgat tatcattggc ggtggagcaa caggcgcagg gatcgcccgc 540

gactgcgcgc tgcgcggttt acgtgtcatt ctggtggagc ggcatgatat agcgaccggc 600

gcgaccgggc gtaaccacgg gctgctgcac agcggcgctc gttatgcggt taccgacgcg 660

gaatccgcgc gcgagtgcat tagtgaaaac cagattttaa aacggatc 708

<210> 26
<211> 21
<212> DNA
<213> Artificial Sequence

<220>
<223> SEQ ID NO. 26 (tumor-specific promotor element of 301glpA)

<400> 26
ccatatttta tttagaggta a 21

<210> 27
<211> 581
<212> DNA
<213> Artificial Sequence

<220>
<223> SEQ ID NO. 27 (310ybaL)

<400> 27
gatcgtatct ttttataaaa cacaaccatt ttttataaac atcctgattg aaattgtcat 60

aaactattac ccggagtttt ggagtccagc aaccaaagga gacggaatgc atcacgctac 120

cccgcttatc accaccattg tcggcggcct tgtgctcgct tttattctcg gcatgattgc 180

caacaaattg cgtatttctc cactggtggg atatctgtta gcgggcgttc tggcgggacc 240

ttttaccccg ggttttgttg cggataccaa actggcgccg gagctggcgg agctcggcgt 300

gattctattg atgttcggcg tcgggctgca tttttcgctg aaggatttga tggcggtaaa 360

gtctatcgcc attcccggcg ctgtcgctca gatagcggtg gcgacgctgc tgggtatggc 420

gctttccgcc gtgctgggat ggtcattaat gaccggcatc gtttttgggc tctgtctgtc 480

tacagccagt accgtcgtcc tgctgcgcgc gcttgaggag cgacaactcc ttgatagcca 540

gcgcgggcaa atcgccatcg gctggctgat tgtcgaagat c 581

<210> 28
<211> 21
<212> DNA
<213> Artificial Sequence

<220>
<223> SEQ ID NO. 28 (tumor-specific promotor element of 310ybaL)

<400> 28
gtatcttttt ataaaacaca a 21

## Description

The present invention relates to bacterial promoter elements which constitute or which are comprised in promoter regions and which confer tumour specificity to the promoter region activity, resulting in transcription of a transgene, which can be a heterologous or a homologous gene, e.g. a human gene or a Salmonella gene, which transgene is functionally arranged downstream of the promoter region at presence of a host bacterium in tumour tissue, while essentially conferring inactivity of the promoter region and no transcription at presence of the host bacterium in non-tumour tissue. Accordingly, the invention relates to bacterial promoter regions containing these tumour specific promoter elements. The tumour specific activity of the promoter elements is that their presence in a promoter region makes the promoter region specifically inducible by the localization of a host bacterial vector in tumour tissue. A bacterial promoter of the invention further confers a significantly reduced activity to a promoter region containing it, preferably no activity at presence of the bacterial vector in non-tumour tissue, i.e. the promoter regions containing a promoter element of the invention are significantly less or essentially not induced at presence of the host bacterial vector in non-tumour tissue. For the purposes of this invention, tumours preferably are solid tissue tumours and/or metastases, e.g. benign and malignant tumours, e.g. solid cancer including metastatic tumour tissue. For use in the treatment of cancer, the invention therefore relates to bacterial vectors containing an expression cassette, wherein a nucleic acid sequence that can be transcribed is arranged under the control of a promoter region comprising or consisting of a tumour specific promoter element. Further, the invention relates to the use of the tumour specific promoter elements in the production of a pharmaceutical composition containing a bacterial vector for use in the treatment of a solid tumour.

### State of the art

Arrach et al. in Cancer Research 68, 4827 - 4832 (2008) describe a method for screening *Salmonella enterica typhimurium* genomic DNA fragments using a reporter gene encoding GFP to identify DNA regions that were enriched in tumour samples of a tumour bearing experimental animal, but not in spleen. Without giving nucleotide sequences, Arrach et al. describe that three promoter sections showed enhanced reporter gene expression when growing in tumour relative to spleen in an *in vivo* test.

The general use of bacterial vectors as a pharmaceutical agent, wherein the promoter region is selected according to its induction pattern is described in WO 99/55364, wherein the arabinose inducible promoter P_{BAD} is used for control of transgene expression, in Bowers et al. (Gene, 11-18, 2004) using the P_{BAD} promoter for control of plasmid copy number in a bacterial vector, and in Wong et al. (Gene 117-186, 2003) using a xylose inducible promoter for tight regulation of the expression of an essential gene in *Haemophilus influenzae.*

Lößner et al. (Expert Opinion on Biological Therapy, 157-168, 2004) describe DNA transfer for gene therapy and vaccination by bacterial vectors. Dietrich et al. (Current Drugs, 10-19, 2003) describe live attenuated bacterial vaccines. WO 2006/079790 describes gut bacteria that comprise a promoter which is induced by presence of a dietary factor to produce a biologically active protein. WO 99/55364 describes the production of antigenic MxiM protein of Shigella for use as a vaccine, *inter alia* by expression under the control of inducible promoters P_{BAD} and P_{LAC}. WO 2006/048344 describes a bacterial vector containing an expression cassette for a transgene under the control of a saccharide inducible promoter for use in tumour therapy, in which the promoter is induced by administration of the inductor saccharide concurrent to or separate from the administration of the bacterial vector.

King et al. in Human Gene Therapy 1225-1233 (2002) describe an attenuated Salmonella vector containing a gene encoding a thioredoxin-cytosine desaminase fusion protein, cloned into a commercial vector designated pTrc99A. The bacterial vector was administered to tumour-bearing mice and expressed the pro-drug converting fusion protein at sites of accumulation, i.e. in tumour tissue.

### Objects of the invention

It is an object of the invention to provide nucleotide sequences conferring tumour specificity to a promoter region, and preferably ensuring essentially no induction of promoter activity in non-tumour tissue, e.g. in spleen. A further object of the invention is to provide bacterial vectors containing the tumour specific promoter elements in an expression cassette to control the tumour specific induction of an orf arranged within the expression cassette.

### General description of the invention

The invention achieves the above-mentioned objects by providing nucleic acid sequences which confer tumour specific promoter activity with little or no promoter activity in non-tumour tissue, e.g. in spleen, when the promoter sequences are contained in or constitute the promoter region of an expression cassette contained in a bacterial vector. For example, the tumour specific promoter elements of the invention can constitute or be comprised in a promoter region of an expression cassette to control transcription of an orf, e.g. a transgene under the control of the promoter region. The tumour specific promoter elements have a modulating effect when present in a promoter region to confer activity to the promoter region at presence of the bacterium comprising the nucleotide sequence within tumour tissue, and preferably conferring inactivity to a promoter region when the bacterium is present in non-tumour tissue like spleen.

Further, the invention provides bacterial vectors containing an expression cassette having at least one of the promoter elements of the invention to control the transcription of an orf, e.g. a transcribable nucleotide sequence like a transgene, under the control of a promoter region comprising or consisting of a promoter element of the invention, which bacterial vector is suitable for use as a pharmaceutical composition, e.g. in the treatment of tumours.

Accordingly, the invention also provides the use of the promoter elements in the production of a pharmaceutical composition containing a bacterial vector, wherein the vector contains an expression cassette, wherein an orf, e.g. a transgene, is functionally arranged under the control of a promoter region containing or consisting of a promoter element according to the invention. Further, the invention provides pharmaceutical compositions comprising the bacterial vector of the invention for use in the medical treatment of a tumour, as well as a process for tumour treatment of a mammal, especially a human, bearing a tumour, the process comprising the administration of the bacterial vector to the mammal.

The bacterial vectors comprising an expression cassette, in which the promoter region consists of or comprises a tumour specific promoter element, are preferably part of a pharmaceutical composition for use in tumour therapy.

In general, nucleotide sequences are given in 5' to 3'.

From a number of pre-selected DNA sections, the promoter elements of the invention have been deduced, which are characterized by their tumour specificity of inducing transcription of a transgene functionally linked downstream to a promoter region containing a tumour specific promoter element, and by the relative inactivity of a promoter region containing the tumour specific promoter element at presence of the bacterial vector in a non-tumour tissue, e.g. in spleen, for induction of transcription of a transgene under their control Therefore, the individual tumour specific promoter elements contained in each of the DNA sections having promoter activity are presently presumed to confer the tumour specificity of promoter activity of the DNA sections.

The tumour specific promoter elements of the invention correspond to or are represented by YHNYDTKTTWTTWANASRWAM (SEQ ID No. 1), wherein non-standard nucleotides are represented as follows: R is G or A, Y is T or C, M is A or C, K is G or T, W is A or T, S is G or C, B is T or G or C, V is A or G or C, H is A or T or C, D is A or T or G, and N is A or T or G or C.

The motif of the promoter element corresponding to SEQ ID No. 1 is closest contained in the best matching motif of SEQ ID No. 2: CAGTATTTTATTTAAAGGTAA

From the nucleic acid sections identified to contain a tumour specific promoter element of the invention, CCATATTTTATTTARAGGTAA (SEQ ID No. 3), with R = A or G, could be inferred from SEQ ID No. 2. In one clone, the following tumour specific promoter element was identified: CCATATTTTATTTAGAGGTAA (SEQ ID No. 4). From the analysis of DNA sections with tumour specific promoter activity, SEQ ID No. 4 was found to be the closest one to the sequences corresponding to SEQ ID Nos. 1 or 2.

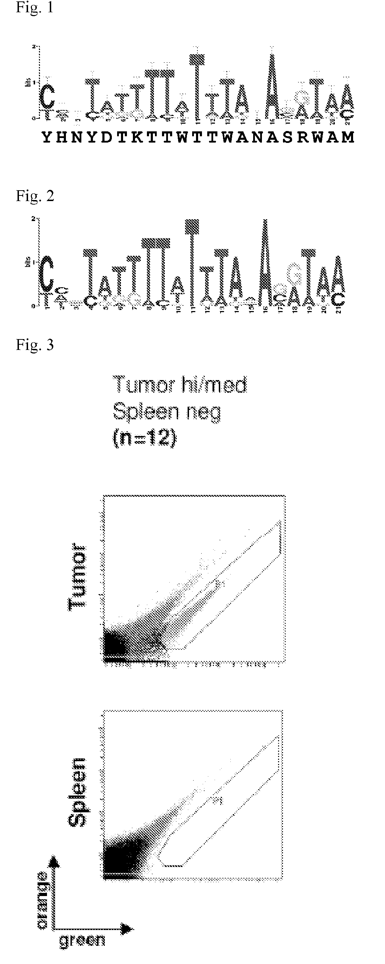

The sequence motif of the tumour specific promoter element is represented in Figure 1 with the Y-axis indicating the incidence of the respective base at the position indicated on the X-axis.

In Figure 2, the sequence motif for the tumour specific promoter element is shown with the relative presence of natural nucleotides, corresponding to SEQ ID No. 2.

The tumour specific promoter elements of the invention, i.e. nucleotide sequences conferring tumour specificity of promoter activity to a promoter, are preferably comprised in a promoter region functionally arranged in 5' to a transgene, e. g. in an expression cassette containing a transgene. Accordingly, the tumour specific promoter elements of the invention are preferably comprised in a promoter region, or the promoter elements of the invention are promoter regions, which are in functional arrangement to a coding sequence, e.g. a transgene, conferring tumour specific transcription of the transgene to the promoter region, e.g. transcription of the transgene from the expression cassette containing the tumour specific promoter moment functionally linked to the transgene.

The specificity of the promoter elements of the invention to be tumour specific, i.e. to initiate transcription of a nucleotide sequence functionally linked to the promoter comprising or consisting of a tumour specific promoter element has been selected for by a selection method comprising at least three, preferably four, consecutive screening steps. By this method, tumour specific promoter elements could be selected that do not initiate transcription of the transgene in the spleen.

For the purpose of the invention, the term transgene comprises both heterologous and homologous genes, e.g. of the human tumour patient or of the bacterial vector. Preferred transgenes are nucleic acid sequences encoding a cytokine, e.g. selected from interleukins, preferably selected from IL-2,IL-4, IL-12, IL-21, IFN-λ, IFN-α, IFN-β, and IFN-γ, GM-CSF, TNF-α, TGF-β, nucleic acid sequences encoding an angiogenesis inhibitor, e.g. selected from thrombospondin-1, endostatin, and angiopoietin-2, nucleic acid sequences encoding a bacterial toxin, e.g. selected from, shiga-like toxin, α-toxin and/or Parton-Valentine leukocidin of *Staphylococcus aureus,* and nucleic acid sequences encoding a prodrug converting enzyme, e.g. nucleic acid sequences encoding carboxypeptidase G2, and/or encoding purine-deoxynucleoside phosphorylase, and/or nucleic acid sequences encoding genes such as colicin, cytolysine and/or cytosine desaminase.

Bacterial vectors are selected among Gram-positive bacteria, preferably among Gram-negative bacteria, most preferably attenuated invasive bacteria. The bacterial vectors of the invention are genetically manipulated to contain an expression cassette containing a coding sequence for a transgene under the control of a bacterial promoter region containing a tumour specific promoter element of the invention. Preferably, the expression cassette is integrated into the bacterial genome. Less preferred, the expression cassette is contained in a plasmid present within the bacterial vector.

The gram-negative bacteria are for example *E. coli, Salmonella* spp., e.g. *Salmonella enterica* serovar Typhimurium, like strain SL7207, e.g. *Salmonella enterica* serovar Typhi, like strain Ty21 a, *Shigella* spp., *Yersinia* spp., and *Vibrio cholerae.* Examples for gram-positive bacteria are *Bacillus* spp., e.g. *Bacillus subtilis, Clostridium* spp., *Listerium monocytogenes,* and *Mycobacterium* spp., e.g. strain BCG. Commensal bacteria are for example *E. coli, Lactobacillus* spp., *Lactococcus* spp., and *Streptococcus gordonii.*

Within the terms of this disclosure, the expression "attenuated invasive bacteria" especially refers to attenuated strains of *E. coli, Listerium monocytogenes, Salmonella enterica* serovar. *typhimurium, Shigella flexneri, Yersinia pseudotuberculosis,* and attenuated strains of further invasive bacteria as well as of the non-invasive bacterium *Vibrio cholerae.* The reason for including *Vibrio cholerae* for the purposes of this disclosure within the term "invasive bacteria" is that for *Vibrio cholerae* attenuated strains exist, which have been demonstrated to accumulate within tumor tissue (Nature Biotechnology, Volume 22, No 3, March 2004). With reference to the affinity of *Vibrio cholerae* to tumours, this is a property shared with at least some invasive bacteria, making them useful within the present invention.

In the context of this disclosure, the classification of bacteria useful as the bacterial vector components of the invention as invasive or non-invasive includes the possibility for some bacteria to colonize organ specific tissue intracellularly and extracellularly. This applies for example to Salmonella, that colonize tumour tissue also in an extracellular state.

Further, invasive bacteria can be used to generate the vector component of the invention which originally are non-invasive bacteria but which have been rendered invasive by genetic manipulation, e.g. by introduction of coding sequences for invasion promoting factors, e.g. the invasin gene (inv) derived from *Yersinia pseudotuberculosis,* e.g. by expression in *E. coli.*

Further exemplary bacteria suitable for producing the bacterial vector according to the invention are: *Agrobacterium* e.g. *Agrobacterium tumefaciens; Bacillus* e.g. *Bacillus cereus, Bacillus subtilis, Bacillus thuringiensis, Bacillus weihenstephanensis; Bartonella* e.g. *Bartonella henselae, Bartonella schoenbuchensis; Bdellovibrio* e.g. *Bdellovibrio bacteriovorus, Bdellovibrio starrii, Bdellovibrio stolpii; Bifidobacterium* e.g. *Bifidobacterium adolescentis, Bifidobacterium bifidum, Bifidobacterium lactis, Bifidobacterium longum; Bordetella* e.g. *Bordetella pertussis; Borrelia* e.g. *Borrelia burgdoiferi; Brucella* e.g. *Brucella abortus, Brucella bronchiseptica; Burkholderia* e.g. *Burkholderia cenocepacia, Burkholderia fungorum, Burkholderia mallei, Burkholderia pseudomallei; Campylobacter* e.g. *Campylobacter fecalis, Campylobacter pylori, Campylobacter sputorum; Chlamydia* e.g. *Chlamydia pneumoniae, Chlamydia psittaci, Chlamydia trachomatis; Clostridium* e.g. *Clostridium difficile, Clostridium novyi, Clostridium oncolyticum, Clostridium perfringens, Clostridium sporogenes, Clostridium tetani; Corynebacterium* e.g. *Cornebacterium diphtheriae, Corynebacterium glutamicum, Corynebacterium jeikeium; Edwardsiella* e.g. *Edwardsiella hoshinae, Edwardsiella ictaluri, Edwardsiella tarda; Enterobacter* e.g. *Enterobacter aerogenes, Enterobacter cloacae, Enterobacter sakazakii; Enterococcus* e.g. *Enterococcus avium, Enterococcus faecalis, Enterococcus faecium, Enterococcus gallinarum; Escherichia* e.g. *Escherichia coli; Eubacterium* e.g. *Eubacterium lentum, Eubacterium nodatum, Eubacterium timidum; Helicobacter* e.g. *Helicobacter pylori; Klebsiella* e.g. *Klebsiella oxytoca, Klebsiella pneumoniae; Lactobacillus* e.g. *Lactobacillus bulgaricus, Lactobacillus casei, Lactobacillus delbrueckii, Lactobacillus plantarum; Lactobacterium* e.g. *Lactobacterium fermentum; Lactococcus* e.g. *Lactococcus lactis, Lactococcus plantarum; Legionella* e.g. *Legionella pneumophila; Listeria* e.g. *Listeria innocua, Listeria ivanovii, Listeria monocytogenes; Microbacterium* e.g. *Microbacterium arborescens, Microbacterium lacticum; Mycobacterium* e.g. *Bacille Calmette-Guérin (BCG), Mycobacterium avium, Mycobacterium bovis, Mycobacterium paratuberculosis, Mycobacterium tuberculosis; Neisseria* e.g. *Neisseria gonorrhoeae, Neisseria lactamica, Neisseria meningitidis; Pasteurella* e.g. *Pasteurella haemolytica, Pasteurella multocida; Salmonella* e.g. *Salmonella bongori, Salmonella enterica* ssp.; *Shigella* e.g. *Shigella dysenteriae, Shigellaflexneri, Shigella sonnei; Staphylococcus e.g. Staphylococcus aureus, Staphylococcus lactis, Staphylococcus saprophyticus; Streptococcus* e.g. *Streptococcus gordonii, Streptococcus lactis, Streptococcus pneumoniae, Streptococcus pyogenes, Streptococcus salivarius; Treponema* e.g. *Treponema denticola, Treponema pallidum; Vibrio* e.g. *Vibrio cholerae; Yersinia* e.g. *Yersinia enterocolitica, Yersinia pseudotuberculosis,* including S1-strains devoid of Hfr factors and of pili of these bacteria, especially S1-strains of *E. coli.*

### Detailed description of the invention

The invention is now described in greater detail with reference to the figures, wherein
- Figure 1 shows a graph indicating the relative prevalence of nucleotides in the motif of the tumour specific promoter element,
- Figure 2 is a graph according to Figure 1 with the best matching nucleotide sequence, and
- Figure 3 shows FACS measurement results of reporter gene expression from bacterial vectors of the invention in tumour and spleen, respectively.

In detail, a library of genome fragments obtained from *Salmonella typhimurium* was generated by arranging genome fragments in 5' to a reporter gene devoid of a promoter. The reporter gene used was gfp-ova according to Bumann, Mol. Microbiol., 1269 - 1283 (2002). The reporter gene product is short-lived in order to prevent accumulation of protein in bacterial cells that could occur by induction from a weak promoter, which was not intended to be isolated. According to standard procedures, the expression cassettes containing the reporter gene and containing, in 5' to the reporter gene, a genomic fragment, was transformed into *Salmonella typhimurium* SL7207 to generate the bacterial library.

An aliquot of the bacterial library was injected directly, i.e. intra-tumourally, into an established subcutaneous CT26 tumour in a mouse in order to avoid a selection of bacteria by migration into the tumour, that was e.g. observed for systemic application of bacteria.

One day after intra-tumoural infection, the tumour was removed and homogenized. Homogenate was sorted by FACS using two colour flow-cytometry to distinguish reporter gene expressing bacteria from autofluorescent debris.

For this first FACS selection, a very broad region was collected to enrich for GFP-OVA-positive clones, in which presence of the reporter gene product indicates the content of a tumour specific promoter element. Collected bacteria were grown overnight on LB agar plates. Of these selected clones, suspensions were made in LB medium. Aliquots of these bacteria were used for a second intra-tumoural infection as described above, and FACS selection of reporter - positive clones from tumour homogenate. For the second FACS selection, more restrictive settings were used in order to select for clones with high expression. The selected bacteria were plated onto agar plates and grown overnight.

The colonies were scraped off the plates and resuspended and then used for infection of a tumour-free mouse i.v. in order to select for GFP-OVA-negative clones from spleen. One day after infection, the spleen was removed and homogenized. From the homogenate, bacteria that were negative for the reporter gene expression were selected by FACS sorting. In this way, it was ensured that promoter elements that had been selected before for their activity in solid tumour tissue, would not show activity in spleen, which organ was taken as a representative for non-tumour tissue.

Finally, bacteria selected from the FACS sorting as reporter-negative in spleen, were grown on LB-plates and resuspended collectively. An aliquot of the bacterial suspension was used for infection of a tumour bearing mouse i.v. As described above, the tumour was removed on the next day and homogenized. From the homogenate, strong expression of reporter gene was used by restricting the settings of FACS to select for GFP-OVA-high expressing clones. As a result, about 3,000 colonies could be plated from the selected fraction of bacteria.

Inserts from bacterial colonies that were grown of the finally selected bacterial fraction were sequenced. For further selecting bacterial clones for inserts driving transcription of the reporter gene in a solid tumour, but essentially not inducing transcription of the reporter gene in non-tumour tissue, e.g. in spleen, individual bacterial clones were again used to infect a tumour bearing mouse i.v. Recovery of bacterial vectors from tumour homogenate and spleen, respectively, was followed by FACS analysis of GFP-OVA expression. The clones were selected by choosing high reporter gene expression in tumour, in combination with little or no expression of the reporter gene in spleen.

The multi-step selection method for the tumour specific promoter elements of the invention demonstrates that induction of transgene transcription is controlled to be tumour specific in combination with essentially no activation of transgene transcription in a non-tumour environment, e.g. in spleen.

In the individual clones, the following tumour specific promoter elements were identified: ttttgtttaaaaaaaatacag (SEQ ID No. 6), ttacctctaaataaaatatgg (SEQ ID No. 8), cactgtgattttttgaggtaa (SEQ ID No. 10), ttacgtgtaaaaaaacaaatg (SEQ ID No. 12), ttacttttaaaaaaccaactg (SEQ ID No. 14), tctcattttttctcaacgtga (SEQ ID No. 16), gcacgttttaataaattgggg (SEQ ID No. 18), cagcattttgtgtataaatta (SEQ ID No. 20), taatctttatatgaaataaga (SEQ ID No. 22), ttgtatgttatttgtacaaac (SEQ ID No. 24), ccatattttatttagaggtaa (SEQ ID No. 26), gtatctttttataaaacacaa (SEQ ID No. 28).

Preferably, a promoter region contains a tumour specific promoter element in a distance of 10 to 600 nucleotides in 5' to the ribosome binding site of an expression cassette containing an orf (open reading frame nucleotide sequence) of a transgene.

Preferably, the promoter regions containing the tumour specific promoter elements are SEQ ID No. 5 for SEQ ID No. 6, SEQ ID No. 7 for SEQ ID No. 8, SEQ ID No. 9 for SEQ ID No. 10, SEQ ID No. 11 for SEQ ID No. 12, SEQ ID No. 13 for SEQ ID No. 14, SEQ ID No. 15 for SEQ ID No. 16, SEQ ID No. 17 for SEQ ID No. 18, SEQ ID No. 19 for SEQ ID No. 20, SEQ ID No. 21 for SEQ ID No. 22, SEQ ID No. 23 for SEQ ID No. 24, SEQ ID No. 25 for SEQ ID No. 26, and SEQ ID No. 27 for SEQ ID No. 28, respectively.

### Example: Bacterial vector for tumour specific expression of a transgene

As a control experiment for expression of the transgene in tumour only, bacterial *Salmonella typhimurium* clones harbouring the tumour specific promoter elements of the invention as nucleic acid sequences integrated into the expression cassette containing the reporter gene gfp-ova were used to intravenously infect BALB/c mice bearing CT26 tumours. 24 hours post infection, tumours, spleens and livers were removed, homogenized and prepared for FACS analysis. As used during the selection method, a volume of 250 µL homogenate in PBS was measured by FACS. It could be shown that transgene expression was obtained for the promoter elements of the invention in tumour tissue, whereas essentially no transgene expression was determined for spleen and liver.

In all animal experiments, generally 6- to 8-week-old female BALB/c mice were used. Injections were subcutaneously at the abdomen with 5 x 10⁵ CT26 cells. For infection with bacterial vectors, mice that had developed tumours of approximately 4-7 mm diameter were injected with about 5x10⁶ CFU of the bacterial vector in suspension in phosphate buffered saline (PBS). For FACS measurements, homogenates were diluted 1: 10 for spleen and liver homogenates, and 1:100 for tumours, respectively, in 0.1% v/v Triton-X100 / PBS containing 2 mM EDTA and filtered using a 30 µm Celltrix filter. Sorting or analysis by FACS was made on a FACSAria or a LSRII (Becton Dickinson, USA) flow cytometer.

An exemplary result for a clone of *S. typhimurium* containing the gfp-ova gene in 3' to a promoter region containing a promoter element of the invention is shown in Figure 3 for the tumour homogenate and the spleen homogenate, respectively. The fluorescence of orange against green was measured for discriminating autofluorescent cellular debris, which gives a higher orange-to-green fluorescence emission ratio compared to bacterial vectors showing reporter gene expression (gated in P1). The inserted field circumscribes the events that could be assigned to high tumour specificity of induction of reporter gene transcription, in combination with essentially no induction of reporter gene transcription in spleen.

This example demonstrates that the promoter elements of the invention, when comprised in a promoter region of an expression cassette, result in the transcription of the orf which is functionally linked to the promoter region at presence of the bacterial vector in tumour tissue, whereas at presence of the bacterial vector in spleen, essentially no transcription is effected.

## Claims

1. Pharmaceutical composition suitable for medical use against tumours, the composition comprising a bacterial vector containing an expression cassette comprising a transgene encoding nucleotide sequence functionally linked to a bacterial promoter, **characterized in that** the bacterial promoter comprises or consists of a promoter element having a nucleotide sequence corresponding to YHNYDTKTTWTTWANASRWAM (SEQ ID No. 1), wherein non-standard nucleotides are represented as follows: R is G or A, Y is T or C, M is A or C, K is G or T, W is A or T, S is G or C, B is T or G or C, V is A or G or C, H is A or T or C, D is A or T or G, and N is A or T or G or C.

2. Pharmaceutical composition according to claim 1, wherein the nucleotide sequence of the promoter element corresponds to SEQ ID No. 3.

3. Pharmaceutical composition according to claim 1, wherein the nucleotide sequence of the promoter element corresponds to SEQ ID No. 4.

4. Pharmaceutical composition according to claim 1, wherein the nucleotide sequence of the promoter element is selected from the group consisting of SEQ ID No. 6, SEQ ID No. 8, SEQ ID No. 10, SEQ ID No. 12, SEQ ID No. 14, SEQ ID No. 16, SEQ ID No. 18, SEQ ID No. 20, SEQ ID No. 22, SEQ ID No. 24, SEQ ID No. 26, and SEQ ID No. 28.

5. Pharmaceutical composition according to one of the preceding claims, wherein the nucleotide sequence of the promoter element is comprised in a sequence selected from the group consisting of SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, and SEQ ID No. 27.

6. Pharmaceutical composition according to one of the preceding claims, wherein the transgene is selected from the group comprising genes of human or bacterial origin, pro-drug converting enzyme genes, genes encoding interleukins, IL-2, IL-12, IL-21, IFN-λ, IFN-α, IFN-β, and IFN-γ, GM-CSF, TNF-α, and/or TGF-β, nucleic acid sequences encoding an angiogenesis inhibitor, thrombospondin-1, endostatin, and/or angiopoietin-2, nucleic acid sequences encoding a bacterial toxin, colicin, shiga-like toxin, α-toxin and/or Parton-Valentine leukocidin of *Staphylococcus aureus,* and nucleic acid sequences encoding cytosine deaminase, carboxypeptidase G2, and/or purine-deoxynucleoside phosphorylase.

7. Use of a bacterial promoter for the production of a pharmaceutical composition for medical use against solid tumours, the pharmaceutical composition comprising a bacterial vector containing an expression cassette comprising a transgene encoding nucleotide sequence which is functionally linked to a bacterial promoter, **characterized in that** the bacterial promoter comprises or consists of a promoter element having a nucleotide sequence corresponding to YHNYDTKTTWTTWANASRWAM (SEQ ID No. 1), wherein non-standard nucleotides are represented as follows: R is G or A, Y is T or C, M is A or C, K is G or T, W is A or T, S is G or C, B is T or G or C, V is A or G or C, H is A or T or C, D is A or T or G, and N is A or T or G or C .

8. Use according to claim 7, **characterized in that** the bacterial promoter is used for the production of a pharmaceutical composition comprising a bacterial vector, in which the nucleotide sequence encoding the transgene is essentially not transcribed in spleen or liver.

9. Use according to claim 7 or 8, wherein the nucleotide sequence of the promoter element is selected from the group consisting of SEQ ID No. 3, SEQ ID No. 4, SEQ ID No. 6, SEQ ID No. 8, SEQ ID No. 10, SEQ ID No. 12, SEQ ID No. 14, SEQ ID No. 16, SEQ ID No. 18, SEQ ID No. 20, SEQ ID No. 22, SEQ ID No. 24, SEQ ID No. 26, and SEQ ID No. 28.

10. Use according to one of claims 7 to 9, wherein the nucleotide sequence of the promoter element is comprised in a sequence selected from the group consisting of SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, and SEQ ID No. 27.

11. Use according to one of claims 7 to 10, wherein the transgene is selected from the group comprising homologous genes, pro-drug converting enzyme genes, genes encoding interleukins, IL-2, IL-12, IL-21, IFN-λ, IFN-α, IFN-β, and IFN-γ, GM-CSF, TNF-α, and/or TGF-β, nucleic acid sequences encoding an angiogenesis inhibitor, thrombospondin-1, endostatin, and/or angiopoietin-2, nucleic acid sequences encoding a bacterial toxin, colicin, shiga-like toxin, α-toxin and/or Parton-Valentine leukocidin of *Staphylococcus aureus,* and nucleic acid sequences encoding a prodrug converting enzyme, cytosine deaminase, carboxypeptidase G2, and/or purine-deoxynucleoside phosphorylase.

12. Method for the production of a pharmaceutical composition comprising a bacterial vector for medical application against solid tumours, **characterized by** genetically manipulating a bacterial vector by introducing a nucleotide construct containing an expression cassette for a transgene, which expression cassette contains a bacterial promoter comprising or consisting of a promoter element having a nucleotide sequence corresponding to YHNYDTKTTWTTWANASRWAM (SEQ ID No. 1), wherein non-standard nucleotides are represented as follows: R is G or A, Y is T or C, M is A or C, K is G or T, W is A or T, S is G or C, B is T or G or C, V is A or G or C, H is A or T or C, D is A or T or G, and N is A or T or G or C.

13. Method according to claim 12, **characterized in that** the nucleotide sequence of the promoter element is selected from the group consisting of SEQ ID No. 3, SEQ ID No. 4, SEQ ID No. 6, SEQ ID No. 8, SEQ ID No. 10, SEQ ID No. 12, SEQ ID No. 14, SEQ ID No. 16, SEQ ID No. 18, SEQ ID No. 20, SEQ ID No. 22, SEQ ID No. 24, SEQ ID No. 26, and SEQ ID No. 28.

14. Method according to claim 12, **characterized in that** the nucleotide sequence of the promoter element is comprised in a sequence selected from the group consisting of SEQ ID No. 5, SEQ ID No. 7, SEQ ID No. 9, SEQ ID No. 11, SEQ ID No. 13, SEQ ID No. 15, SEQ ID No. 17, SEQ ID No. 19, SEQ ID No. 21, SEQ ID No. 23, SEQ ID No. 25, and SEQ ID No. 27.

15. Method according to one of claims 12 to 14, wherein the transgene is selected from the group comprising homologous genes, pro-drug converting enzyme genes, genes encoding interleukins, IL-2, IL-12, IL-21, IFN-λ, IFN-α, IFN-β, and IFN-γ, GM-CSF, TNF-α, and/or TGF-β, nucleic acid sequences encoding an angiogenesis inhibitor, thrombospondin-1, endostatin, and/or angiopoietin-2, nucleic acid sequences encoding a bacterial toxin, colicin, shiga-like toxin, α-toxin and/or Parton-Valentine leukocidin of *Staphylococcus aureus,* and nucleic acid sequences encoding a prodrug converting enzyme, cytosine deaminase, carboxypeptidase G2, and/or purine-deoxynucleoside phosphorylase.
